# EUROPEAN PATENT APPLICATION

(11) **EP 4 371 486 A1**
(43) Date of publication of application: **22.05.2024**
(21) Application number: 22858748.1
(22) Date of filing: 17.08.2022
(51) Int. Cl.: A61B 5/327, A61B 5/346, A61B 5/28, A61B 5/00, G16H 50/20

(54) **SIMULTANEOUS ELECTROCARDIOGRAM GENERATION METHOD BASED ON 2-LEAD NON-SIMULTANEOUS ELECTROCARDIOGRAMS**

(30) Priority: 17.08.2021 KR 20210107773
(71) Applicant: Medicalai Co., Ltd., Seoul 06302 (KR)
(72) Inventor: KWON, Joon Myoung, Seoul 06629 (KR)
(74) Representative: Isarpatent
(86) International application number: PCT/KR2022/012283
(87) International publication number: WO 2023/022511

(57) **Abstract**

Disclosed herein is a method of generating synchronous electrocardiograms using a synchronous electrocardiogram generation apparatus, the method including: acquiring a plurality of pieces of electrocardiogram data, measured asynchronously, from a 1-lead electrocardiogram device; classifying the plurality of pieces of acquired electrocardiogram data into base electrocardiogram data and reference electrocardiogram data, and converting the reference electrocardiogram data into a plurality of pieces of reference unit electrocardiogram data by dividing the reference electrocardiogram data on a per-bit basis; extracting a first characteristic value of a time unrelated to electrocardiogram leads from the base electrocardiogram data, and extracting second characteristic values of times unrelated to electrocardiogram leads from the plurality of pieces of reference unit electrocardiogram data; and setting base points in the base electrocardiogram data, and generating a synchronized virtual electrocardiogram by arranging the plurality of pieces of reference unit electrocardiogram data based on the set base points.

## Description

### Technical Field

The present invention relates to a synchronous electrocardiogram generation method based on 2-lead asynchronous electrocardiograms, and more specifically to a method of generating synchronous electrocardiogram information from 2-lead asynchronous electrocardiogram information, measured at different times, using a rule-based algorithm.

### Background Art

An electrocardiogram is a graphical record of electrical potentials related to heartbeat on the surface of the human body. Such electrocardiograms include not only standard 12-lead electrocardiograms but also exercise stress electrocardiograms and Holter electrocardiograms. Electrocardiograms are used for the examination and diagnosis of circulatory diseases, and have the advantages of being simple, relatively inexpensive, non-invasive, and easily recorded repeatedly.

As for a standard 12-lead electrocardiogram used in hospitals, six electrodes are attached to the front of the chest, three electrodes (four electrodes when a ground electrode is included) are attached to the limbs, 12-lead information is all collected and combined, and then a disease is diagnosed. A 12-lead electrocardiogram records electrical potentials of the heart in 12 electrical directions centered on the heart. Through this, the disease of the heart confined to one area can be accurately diagnosed.

However, it is difficult to measure a 12-lead electrocardiogram at home or in everyday life because the chest must be exposed to attach chest electrodes to take a 12-lead electrocardiogram and it is difficult for the general public to attach nine electrodes (three limb electrodes and six chest electrodes) to correct locations. In addition, it is difficult to use a 12-lead electrocardiogram for real-time monitoring because it is difficult to move after the attachment of ten electrodes.

Recently, there has been proposed a technology for measuring the side leads of a plurality of electrocardiograms using a 1-lead electrocardiogram device such as a smart watch. However, when electrocardiograms are measured using the 1-lead electrocardiogram device, a problem arises in that the accurate state of the heart cannot be determined because the electrocardiograms are not measured at the same time.

A background technology of the present invention is disclosed in Korean Patent Application Publication No. 10-2019-0071815 (published on June 24, 2019).

### Disclosure

### Technical Problem

As described above, the present invention is intended to provide a method of generating synchronous electrocardiogram information from 2-lead asynchronous electrocardiogram information, measured at different times, using a rule-based algorithm.

### Technical Solution

According to an embodiment of the present invention for overcoming the above technical problem, there is provided a method of generating synchronous electrocardiograms using a synchronous electrocardiogram generation apparatus, the method including: acquiring a plurality of pieces of electrocardiogram data, measured asynchronously, from a 1-lead electrocardiogram device; classifying the plurality of pieces of acquired electrocardiogram data into base electrocardiogram data and reference electrocardiogram data, and converting the reference electrocardiogram data into a plurality of pieces of reference unit electrocardiogram data by dividing the reference electrocardiogram data on a per-bit basis; extracting a first characteristic value of a time unrelated to electrocardiogram leads from the base electrocardiogram data, and extracting second characteristic values of times unrelated to electrocardiogram leads from the plurality of pieces of reference unit electrocardiogram data; and setting base points in the base electrocardiogram data, and generating a synchronized virtual electrocardiogram by arranging the plurality of pieces of reference unit electrocardiogram data based on the set base points.

Converting the reference electrocardiogram data into the plurality of pieces of reference unit electrocardiogram data may include converting the reference electrocardiogram data into the plurality of pieces of reference unit electrocardiogram data by extracting peak points of any one waveform of P, Q, R, S and T waves included in the reference electrocardiogram data and extracting data corresponding to a preset time centered on each of the extracted peak points.

Extracting the characteristic values may include extracting a first characteristic value of a time unrelated to the electrocardiogram leads by using the time length of the any one waveform of the P, Q, R, S and T waves included in the base electrocardiogram data and also extracting second characteristic values of times unrelated to the electrocardiogram leads by using the time length of the reference electrocardiogram data.

Generating the synchronized virtual electrocardiogram may include setting the peak points of the any one waveform of the P, Q, R, S and T waves, included in the base electrocardiogram data, as base points and arranging the plurality of pieces of reference unit electrocardiogram data based on the set base points.

Generating the synchronized virtual electrocardiogram may include: extracting reference unit electrocardiogram data having a second characteristic value most similar to the first characteristic value by comparing the first characteristic value of the base electrocardiogram data and the second characteristic values of the reference unit electrocardiogram data; setting the peak point of the any one waveform of the P, Q, R, S and T waves, included in the base electrocardiogram data, as a base point; and arranging the extracted reference unit electrocardiogram data based on the set base point.

Generating the synchronized virtual electrocardiogram may include adding an electrocardiogram, located in the rest period section of a base electrocardiogram or a reference electrocardiogram, between the arranged reference unit electrocardiogram data and another piece of reference unit electrocardiogram data.

### Advantageous Effects

As described above, according to the present invention, a virtual electrocardiogram may be generated by extracting a reference electrocardiogram having a characteristic value most similar to the characteristic value of a base electrocardiogram through the use of the characteristic values of the base electrocardiogram and reference electrocardiograms and also arranging it based on a base point, so that electrocardiograms measured at different times can be synchronized with each other, thereby enabling medical staff to increase the accuracy of reading of electrocardiograms.

### Description of Drawings

FIG. 1 is a block diagram of a synchronous electrocardiogram generation apparatus according to an embodiment of the present invention;
FIG. 2 is a flowchart illustrating a method of generating synchronous electrocardiograms using a synchronous electrocardiogram generation apparatus according to an embodiment of the present invention;
FIG. 3 is an example diagram illustrating step S220 shown in FIG. 2;
FIG. 4 is an example diagram illustrating a method of arranging reference unit electrocardiogram data in step S240 shown in FIG. 2; and
FIG. 5 is an example diagram showing a synchronized virtual electrocardiogram generated in step S240 shown in FIG. 2.

### Mode for Invention

Preferred embodiments according to the present invention will be described in detail below with reference to the accompanying drawings. In this process, the thicknesses of lines or sizes of components shown in the drawings may be shown exaggerated for the clarity and convenience of description.

Additionally, the terms to be described below are terms defined by taking into consideration the functions thereof in the present invention, and may vary depending on the intention or custom of a user or operator. Accordingly, the definitions of these terms should be made based on the content throughout the present specification.

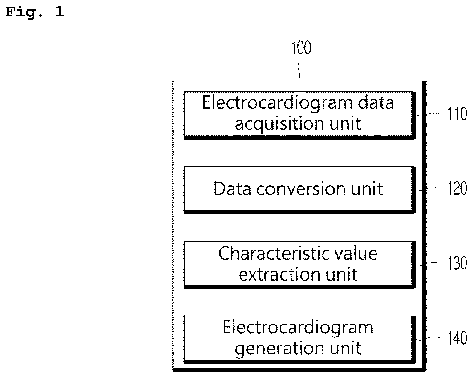

A synchronous electrocardiogram generation apparatus according to an embodiment of the present invention will be described in more detail below using FIG. 1.

FIG. 1 is a block diagram of a synchronous electrocardiogram generation apparatus according to an embodiment of the present invention.

As shown in FIG. 1, a synchronous electrocardiogram generation apparatus 100 according to an embodiment of the present invention includes an electrocardiogram data acquisition unit 110, a data conversion unit 120, a characteristic value extraction unit 130, and an electrocardiogram generation unit 140.

First, the electrocardiogram data acquisition unit 110 acquires a plurality of pieces of electrocardiogram data measured asynchronously.

The data conversion unit 120 classifies the plurality of pieces of electrocardiogram data into base electrocardiogram data and reference electrocardiogram data, and converts the reference electrocardiogram data into a plurality of pieces of reference unit electrocardiogram data.

The characteristic value extraction unit 130 extracts a first characteristic value from the base electrocardiogram data, and extracts second characteristic values from the plurality of pieces of reference unit electrocardiogram data, respectively.

Finally, the electrocardiogram generation unit 140 generates a virtual electrocardiogram synchronized with the base electrocardiogram data by arranging the plurality of pieces of reference unit electrocardiogram data using the first characteristic value of the base electrocardiogram data and the second characteristic values of the plurality of pieces of reference unit electrocardiogram data.

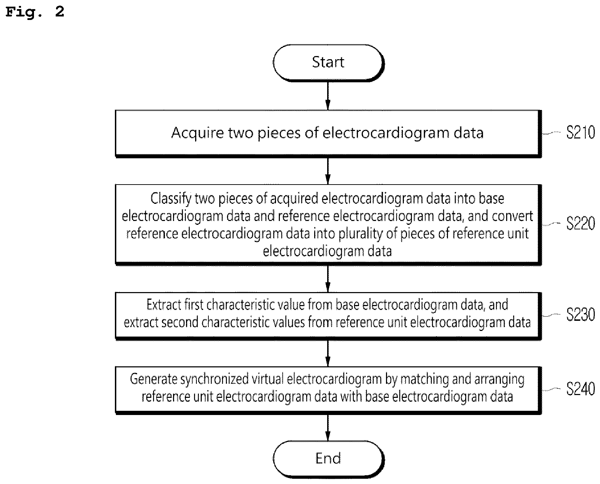

A method of generating synchronous electrocardiograms using the synchronous electrocardiogram generation apparatus 100 according to an embodiment of the present invention will be described in more detail below using FIGS. 2 to 5.

FIG. 2 is a flowchart illustrating a method of generating synchronous electrocardiograms using a synchronous electrocardiogram generation apparatus according to an embodiment of the present invention.

As shown in FIG. 2, the synchronous electrocardiogram generation apparatus 100 according to an embodiment of the present invention acquires two pieces of electrocardiogram data in step S210.

In addition, the electrocardiogram data acquisition unit 110 acquires two pieces of electrocardiogram data from a single-lead electrocardiogram device (not shown). In this case, the two pieces of electrocardiogram data are asynchronous electrocardiogram data.

Next, the data conversion unit 120 classifies the two pieces of acquired electrocardiogram data into base electrocardiogram data and reference electrocardiogram data and converts the reference electrocardiogram data into a plurality of pieces of reference unit electrocardiogram data in step S220.

In other words, the data conversion unit 120 sets any one of the two pieces of acquired electrocardiogram data as base electrocardiogram data and also sets the other piece of electrocardiogram data as reference electrocardiogram data.

Next, the data conversion unit 120 converts the reference electrocardiogram data into a plurality of pieces of reference unit electrocardiogram data using the peak points of waveforms constituting the reference electrocardiogram data.

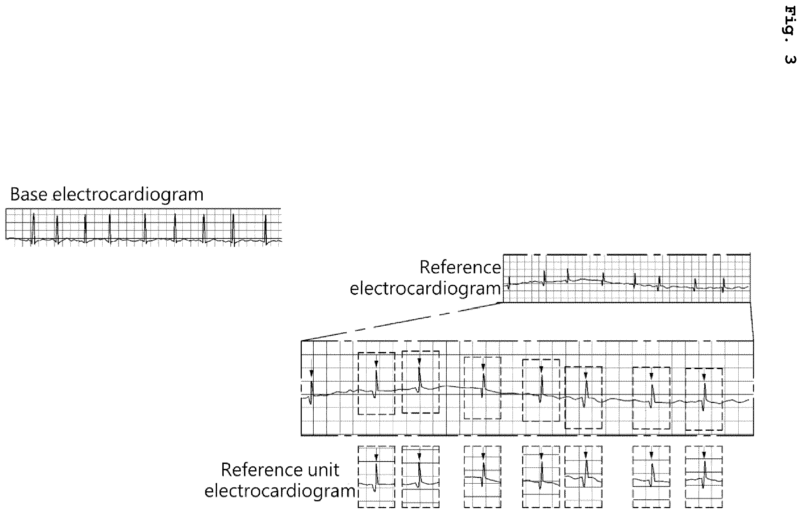

FIG. 3 is an example diagram illustrating step S220 shown in FIG. 2.

As shown in FIG. 3, the data conversion unit 120 extracts peak points from the waveforms included in the reference electrocardiogram data. For example, the data conversion unit 120 extracts the peak points (the black arrows) of the R waves of the reference electrocardiogram data and converts the reference electrocardiogram data into a plurality of pieces of reference unit electrocardiogram data at preset predetermined time intervals centered on the extracted peak points.

When step S220 is completed, the characteristic value extraction unit 130 extracts a first characteristic value from the base electrocardiogram data and also extracts second characteristic values from the plurality of pieces of reference unit electrocardiogram data in step S230.

The characteristic value extraction unit 130 extracts the first characteristic value of a time unrelated to electrocardiogram leads by using the time length of any one waveform of the P, Q, R, S, and T waves included in the base electrocardiogram data.

Furthermore, the characteristic value extraction unit 130 extracts the second characteristic values of times unrelated to electrocardiogram leads using the time length of the plurality of pieces of reference unit electrocardiogram data.

Finally, the electrocardiogram generation unit 140 generates a synchronized virtual electrocardiogram by arranging the plurality of pieces of reference unit electrocardiogram data in accordance with the base electrocardiogram data in step S240.

First, the electrocardiogram generation unit 140 compares the first characteristic value of the base electrocardiogram data with the second characteristic values of the plurality of pieces of reference unit electrocardiogram data.

In addition, the electrocardiogram generation unit 140 extracts a second characteristic value similar to the first characteristic value, and arranges reference unit electrocardiogram data corresponding to the extracted second characteristic value by matching the reference unit electrocardiogram data with the base electrocardiogram data.

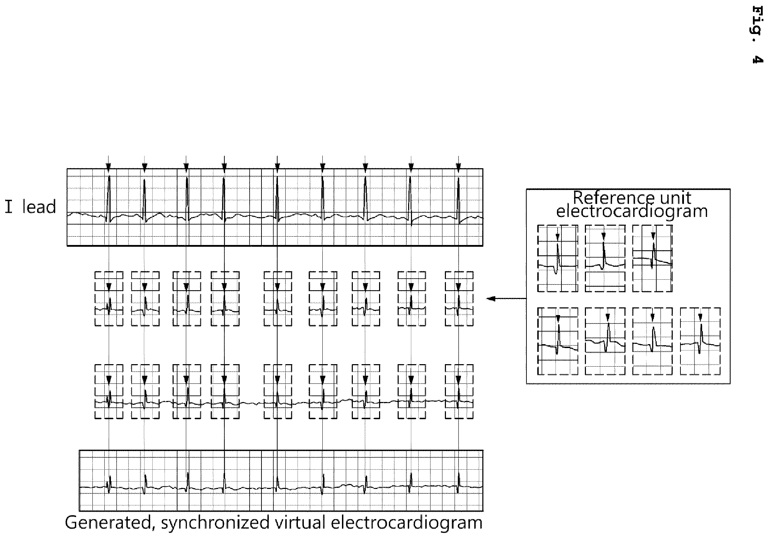

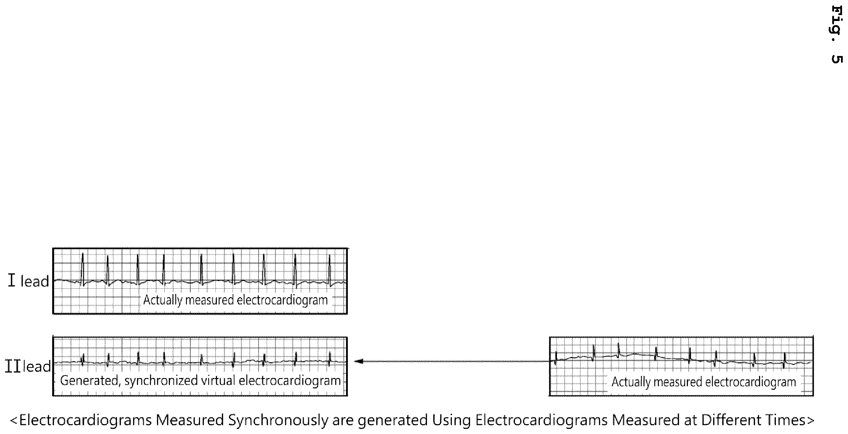

FIG. 4 is an example diagram illustrating a method of arranging reference unit electrocardiogram data in step S240 shown in FIG. 2, and FIG. 5 is an example diagram showing a synchronized virtual electrocardiogram generated in step S240 shown in FIG. 2.

As shown in FIG. 4, the electrocardiogram generation unit 140 arranges corresponding reference unit electrocardiogram data in accordance with each of the base points of waveforms constituting the base electrocardiogram data.

Next, the electrocardiogram generation unit 140 connects the arranged reference unit electrocardiogram data with other reference electrocardiogram data. In this case, the electrocardiogram generation unit 140 connects them by referring to an electrocardiogram in a rest period section included in the base electrocardiogram data or the reference electrocardiogram data or by taking into consideration the start and end point values of the reference unit electrocardiogram data.

Then, as shown in FIG. 5, there is generated a synchronized virtual electrocardiogram that is different from an actually measured electrocardiogram.

As described above, according to the present invention, a virtual electrocardiogram may be generated by extracting a reference electrocardiogram having a characteristic value most similar to the characteristic value of a base electrocardiogram through the use of the characteristic values of the base electrocardiogram and reference electrocardiograms and also arranging it in accordance with a base point, so that electrocardiograms measured at different times can be synchronized with each other, thereby enabling medical staff to increase the accuracy of reading of electrocardiograms.

While the present invention has been described with reference to the embodiments shown in the drawings, these are merely examples. It will be understood by those having ordinary skill in the art to which the corresponding technology pertains that various modifications and other equivalent embodiments may be made therefrom. Therefore, the true technical protection range of the present invention should be determined by the technical spirit of the following patent claims.

## Claims

1. A method of generating synchronous electrocardiograms using a synchronous electrocardiogram generation apparatus, the method comprising:
acquiring a plurality of pieces of electrocardiogram data, measured asynchronously, from a lead electrocardiogram device;
classifying the plurality of pieces of acquired electrocardiogram data into base electrocardiogram data and reference electrocardiogram data, and converting the reference electrocardiogram data into a plurality of pieces of reference unit electrocardiogram data by dividing the reference electrocardiogram data on a per-bit basis;
extracting a first characteristic value of a time unrelated to electrocardiogram leads from the base electrocardiogram data, and extracting second characteristic values of times unrelated to electrocardiogram leads from the plurality of pieces of reference unit electrocardiogram data; and
setting base points in the base electrocardiogram data, and generating a synchronized virtual electrocardiogram by arranging the plurality of pieces of reference unit electrocardiogram data based on the set base points.

2. The method of claim 1, wherein converting the reference electrocardiogram data into the plurality of pieces of reference unit electrocardiogram data comprises converting the reference electrocardiogram data into the plurality of pieces of reference unit electrocardiogram data by extracting peak points of any one waveform of P, Q, R, S and T waves included in the reference electrocardiogram data and extracting data corresponding to a preset time centered on each of the extracted peak points.

3. The method of claim 2, wherein extracting the characteristic values comprises extracting a first characteristic value of a time unrelated to the electrocardiogram leads by using a time length of the any one waveform of the P, Q, R, S and T waves included in the base electrocardiogram data and also extracting second characteristic values of times unrelated to the electrocardiogram leads by using a time length of the reference electrocardiogram data.

4. The method of claim 3, wherein generating the synchronized virtual electrocardiogram comprises setting peak points of the any one waveform of the P, Q, R, S and T waves, included in the base electrocardiogram data, as base points and arranging the plurality of pieces of reference unit electrocardiogram data based on the set base points.

5. The method of claim 3, wherein generating the synchronized virtual electrocardiogram comprises:
extracting reference unit electrocardiogram data having a second characteristic value most similar to the first characteristic value by comparing the first characteristic value of the base electrocardiogram data and the second characteristic values of the reference unit electrocardiogram data;
setting a peak point of the any one waveform of the P, Q, R, S and T waves, included in the base electrocardiogram data, as a base point; and
arranging the extracted reference unit electrocardiogram data based on the set base point.

6. The method of claim 4 or 5, wherein generating the synchronized virtual electrocardiogram comprises adding an electrocardiogram located in a rest period section of a base electrocardiogram or a reference electrocardiogram between the arranged reference unit electrocardiogram data and another piece of reference unit electrocardiogram data.
